# EUROPÄISCHE PATENTSCHRIFT

(11) **EP 1 046 629 B1**
(45) Veröffentlichungstag und Bekanntmachung des Hinweises auf die Patenterteilung: **26.02.2003**
(21) Anmeldenummer: 00106875.8
(22) Anmeldetag: 31.03.2000
(51) Int. Cl.: C07C 51/41, C07C 53/10

(54) **Verfahren zur Herstellung von Iridiumacetat**
Process for preparing iridium acetate
Procédé pour la préparation de l'acétate d'iridium

(30) Priorität: 19.04.1999 DE 19917681
(43) Veröffentlichungstag der Anmeldung: 25.10.2000
(73) Patentinhaber: W.C. Heraeus GmbH & Co. KG, 63450 Hanau (DE)
(72) Erfinder: Kirchner, Sonja, 63628 Bad Soden-Salmünster (DE); Walter, Richard, Dr., 63755 Alzenau (DE)
(74) Vertreter: Kühn, Hans-Christian

(56) Entgegenhaltungen:
- WO-A-96/23757

## Beschreibung

Die Erfindung betrifft ein Verfahren zur Herstellung von Iridiumacetat und die Verwendung des nach dem Verfahren hergestellten Iridiumacetats. Die Erfindung betrifft besonders die Herstellung von Iridiumacetat als Feststoff.

Eine bekannte Verwendung von Iridium und Iridiumverbindungen ist die als Katalysatoren und Katalysatorvorläufer. So werden zum Beispiel nach EP 0 616 997 A1 und EP 0 786 447 A1 Iridium-Katalysatoren bei Carbonylierungsreaktionen eingesetzt; als für diesen Zweck geeignete Iridiumverbindung wird u. a. Iridiumacetat genannt.

Aus dem deutschen Patent 1 127 888 ist es bekannt, Vinylester höherer Carbonsäuren durch Umesterung der Vinylester niederer Carbonsäuren in Gegenwart von Platingruppenmetallsalzen herzustellen. Beispiele für geeignete Platingruppenmetallsalze sind unter anderem die Acetate des Palladiums und des Rhodiums, die aus den in Eisessig gelösten Hydroxiden durch Umsetzung mit Essigsäureanhydrid erhalten werden.

Es ist die Aufgabe der Erfindung, ein Verfahren zur Herstellung von Iridiumacetat, besonders von festem Iridiumacetat, aus gut zugänglichen Chloroverbindungen des Iridiums anzugeben. Das nach dem Verfahren hergestellte Iridiumacetat soll möglichst chloridarm sein und sich besonders für katalytische Zwecke eignen.

Das die Lösung der Aufgabe darstellende Verfahren ist erfindungsgemäß dadurch gekennzeichnet. daß aus einer wässerigen Lösung einer Iridiumchloroverbindung mit einer wässerigen Lösung eines Alkalimetallhydroxids, -carbonats oder -hydrogencarbonats Iridiumhydroxid gefällt, das gefällte Iridiumhydroxid abgetrennt und mit Essigsäure oder einem Essigsäure/Essigsäureanhydrid-Gemisch zu einer Iridiumacetat enthaltenden Lösung umgesetzt und das Iridiumacetat als Feststoff aus der Lösung isoliert wird.

Besonders bewährt hat sich das erfindungsgemäße Verfahren, wenn das Iridiumhydroxid vor der Umsetzung mit Essigsäure oder dem Essigsäure/Essigsäureanhydrid-Gemisch umgefällt wird. Dazu wird das Iridiumhydroxid mit Salpetersäure oder mit Salpetersäure und Wasserstoffperoxid gelöst und aus der erzeugten Lösung durch Zugabe einer wässerigen Lösung eines Alkalimetallhydroxids, -carbonats oder -hydrogencarbonats erneut Iridiumhydroxid gefällt. Die Umfällung kann wiederholt werden, wenn besonders chloridarmes Iridiumacetat erhalten werden soll.

Das Verfahren geht von bekannten Iridiumchloroverbindungen aus. Als gut geeignet haben sich Iridium(III)-chlorid, Iridium(IV)-chlorid, Hexachloroiridium(III)-säure und Hexachloroiridium(IV)-säure erwiesen. Diese Verbindungen können einzeln oder im Gemisch miteinander eingesetzt werden. Bewährt haben sich als Ausgangslösungen wässerige Lösungen der Iridiumchloroverbindungen mit einer Iridium-Konzentration von 2 - 10 %; Iridium-Konzentrationen von 2 - 3 Gewichts-% werden jedoch bevorzugt.

Es hat sich als besonders vorteilhaft erwiesen, zur Fällung des Iridiumhydroxids wässerige Kaliumhydroxid-Lösung (Kalilauge) zu benutzen und einen pH-Wert zwischen 6 und 9, vorzugsweise von etwa 7 - 8, und eine Temperatur von 50 - 90 °C, vorzugsweise von etwa 80 °C, zu wählen. Unter diesen Bedingungen bildet sich ein grobflockiger Niederschlag, der sich gut abtrennen läßt.

Ist der Schritt der Umfällung des Iridiumhydroxids vorgesehen, so wird das Iridiumhydroxid entweder mit Salpetersäure oder mit Salpetersäure und Wasserstoffperoxid gelöst, wobei sich ein Volumenverhältnis von konzentrierter Salpetersäure zu 30 %iger Wasserstoffperoxid-Lösung von etwa 40 zu 1 als günstig erwiesen hat und ein Erwärmen auf bis zu etwa 80 °C vorteilhaft sein kann.

Bei der Umsetzung des Iridiumhydroxids mit Essigsäure oder dem Essigsäure/Essigsäureanhydrid-Gemisch wird ein Molverhältnis von Essigsäure zu Iridium gewählt, das deutlich überstöchiometrisch ist; denn die Essigsäure ist sowohl Reaktionspartner als auch Lösungsmittel für das gebildete Iridiumacetat.

Mit Hilfe des erfindungsgemäßen Verfahrens läßt sich Iridiumacetat in guter Ausbeute herstellen. Charakteristisch für das nach dem Verfahren hergestellte Iridiumacetat ist seine grüne bis dunkelgrüne Farbe, seine gute bis sehr gute Löslichkeit in Wasser, Methanol und Eisessig und ein Zersetzungspunkt oberhalb von 100 °C.

Das nach dem erfindungsgemäßen Verfahren hergestellte Iridiumacetat eignet sich besonders als Katalysator und Katalysatorvorläufer für die homogene Katalyse. Vorteile bietet auch seine Anwendung zum Beschichten und Imprägnieren von anorganischen und organischen Substraten, zum Beispiel bei der Herstellung von Katalysatoren für die heterogene Katalyse.

Zur näheren Erläuterung wird in den folgenden Beispielen die Herstellung von Iridiumacetat nach dem erfindungsgemäßen Verfahren beschrieben. Wasser wird in vollentsalzter Form eingesetzt.

### Beispiel 1

10 g Iridium in Form von Iridium(IV)-chlorid, IrCl₄, werden in 500 ml Wasser gelöst. Die erhaltene dunkelviolette Lösung, die einen pH-Wert von 1,29 besitzt, wird auf 80 °C erwärmt, innerhalb von 1,5 Stunden tropfenweise mit 230 ml 1 n KOH-Lösung bis pH 7 versetzt und dann noch eine Stunde lang gerührt. Das schwarzes, feinkristallines Iridiumhydroxid enthaltende Reaktionsgemisch wird über Nacht stehengelassen; dann wird das Iridiumhydroxid abfiltriert und mit 1,5 I Wasser gewaschen, bis das Filtrat farblos, klar und chloridfrei ist. Der aus Iridiumhydroxid bestehende Filterkuchen wird trockengesaugt und dann in einen 500 ml-Dreihalskolben gegeben und mit 300 ml Eisessig unter Bildung einer schwarzen Suspension versetzt. Die Suspension wird über Nacht unter Rückfluß gekocht. Nach Abkühlen auf Raumtemperatur wird die so erzeugte dunkelgrüne Lösung über ein Glasfaserfilter filtriert und anschließend im Rotationsverdampfer bei etwa 70 °C im Vakuum (Wasserstrahlvakuum) zur Trockne eingedampft. Das gewonnene Produkt wird im Vakuumtrockenschrank bei etwa 50 °C bis zur Gewichtskonstanz getrocknet. Es werden 17,6 g eines pulverförmigen, dunkelgrünen Feststoffes erhalten. Die Ausbeute beträgt 87 %.

### Beispiel 2

5 g Iridium in Form einer Hexachloroiridium(IV)-säure-Lösung werden in einen 250 ml-Kolben gegeben und bei etwa 60 °C im Wasserstrahlvakuum zu einem zähflüssigen Öl eingeengt, das beim Abkühlen auf Raumtemperatur einen dunkelrot-braunen Feststoff bildet. Dann wird der Feststoff in 150 ml Wasser gelöst und die erhaltene dunkelrot-braune Lösung auf etwa 80 °C erwärmt und tropfenweise mit 1n KOH-Lösung bis pH 7,3 versetzt, wobei sich eine blau-schwarze, Iridiumhydroxid enthaltende Suspension bildet. Die warme Suspension wird auf ein Blaubandfilter gegeben; der so abgetrennte Filterkuchen wird mit insgesamt etwa 2500 ml Wasser chloridfrei gewaschen, anschließend trockengesaugt, in einen 250 ml-Dreihalskolben gegeben und mit 150 ml Eisessig versetzt. Die entstandene blau-schwarze Suspension wird im Ölbad auf Siedetemperatur erhitzt und unter Rückflußbedingungen und Rühren etwa 25 Stunden lang bei dieser Temperatur gehalten. Dann werden 50 ml Eisessig zugegeben, und es wird nochmals 2 Stunden lang bei Siedetemperatur gerührt. Nach Abkühlen auf Raumtemperatur wird das Reaktionsgemisch über ein Glasfaserfilter filtriert und dann das so gewonnene klare, dunkelgrüne Filtrat im Rotationsverdampfer bei etwa 70 °C im Wasserstrahlvakuum zur Trockne eingedampft. Der zurückbleibende dunkelgrün-glänzende Feststoff wird im Vakuumtrockenschrank bei etwa 50 °C bis zur Gewichtskonstanz getrocknet und dann im Mörser zerrieben. Es werden 9,8 g feinpulvriges, dunkelgrün-glänzendes Iridiumacetat erhalten; die Ausbeute beträgt 93 %.

### Beispiel 3

25,5 g Iridium in Form von Hexachloroiridium(III)-säure-Lösung werden in ein 2 I-Becherglas gegeben und mit soviel Wasser versetzt, daß das Gesamtvolumen 1000 ml beträgt. Die erhaltene dunkelrote Lösung wird auf 60 °C erwärmt und innerhalb von einer Stunde tropfenweise mit 45 %iger KOH-Lösung versetzt, bis der pH-Wert etwa 8,1 beträgt. Es wird eine blau-graue Suspension erhalten, die nach Abkühlen auf Raumtemperatur über ein Blaubandfilter filtriert wird. Der Filterkuchen wird mit insgesamt 6 I Wasser gewaschen, bis sich im Filtrat kein Chlorid mehr nachweisen läßt. Nach Trockensaugen wird der Filterkuchen in 200 ml konzentrierter Salpetersäure (65 %ig) und 100 ml Wasser gelöst. Die erhaltene Lösung wird eine halbe Stunde lang bei Raumtemperatur gerührt, dann über ein Glasfaserfilter filtriert, auf etwa 65 °C erwärmt und innerhalb einer Stunde tropfenweise mit 45 %iger KOH-Lösung versetzt, bis der pH-Wert 8.3 beträgt. Es entsteht eine blau-schwarze Suspension, die auf ca. 40 °C abgekühlt und dann über ein Blaubandfilter filtriert wird. Es wird mit 5 I Wasser gewaschen, bis das Filtrat chlorid- und nitratfrei ist. Der abgetrennte schwarze Feststoff wird trockengesaugt und dann mit 100 ml Wasser in ein 1 l-Becherglas überführt. Dann werden 200 ml konzentrierte Salpetersäure zugesetzt, wobei eine dunkelblaue Lösung entsteht. Anschließend wird noch 1/2 Stunde lang bei Raumtemperatur gerührt. Dann folgt die Filtration über ein Glasfaserfilter. Die filtrierte Lösung wird in ein Becherglas gegeben, auf etwa 60 °C erhitzt und tropfenweise mit 45 %iger KOH-Lösung bis pH 8,4 versetzt. Die erhaltene blauschwarze Suspension wird auf etwa 40 °C abgekühlt und über ein Blaubandfilter filtriert. Der dadurch gewonnene Filterkuchen wird mit 5 l Wasser gewaschen, bis das Filtrat chlorid- und nitratfrei ist, dann trockengesaugt und mit 200 ml Essigsäureanhydrid in einen 1 l-Dreihalskolben überführt. Nach Zusatz von 200 ml Eisessig wird die schwarze Suspension 18 Stunden lang auf etwa 110 °C erhitzt. Es entsteht eine dunkelgrüne, leicht trübe Lösung, die über ein Glasfaserfilter filtriert wird. Dann wird Wasser in einer Menge von 60 ml zugesetzt und 1 Stunde lang auf etwa 80 °C erhitzt. Die dunkelgrün-braune Lösung wird im Rotationsverdampfer bei etwa 80 °C zur Trockne eingedampft; das erhaltene Produkt wird dann noch bei 60 °C im Vakuumtrockenschrank bis zur Gewichtskonstanz getrocknet. Es werden 49,2 g Iridiumacetat in Form eines dunkelgrün-glänzenden Feststoffes erhalten. Die Ausbeute beträgt 88 %.

### Beispiel 4

70 g Iridium in Form von Hexachloroiridium(IV)säure-Lösung werden in ein Becherglas gegeben und mit soviel Wasser verdünnt, daß das Gesamtvolumen 2 I beträgt. Die erhaltene dunkelrote Lösung wird auf etwa 60 °C erwärmt und unter Rühren tropfenweise mit 45 %iger KOH-Lösung versetzt, bis der pH-Wert etwa 8 beträgt. Es wird eine schwarze Suspension erhalten, die nach Abkühlen auf Raumtemperatur über ein Blaubandfilter filtriert wird. Der Filterkuchen wird zunächst mit 4 kaltem Wasser, dann mit 1 l heißem Wasser und und anschließend mit 5 l kaltem Wasser gewaschen, bis das Filtrat farblos und klar ist und sich kein Chlorid mehr nachweisen läßt. Der schwarze Filterkuchen wird trockengesaugt, in einen 2000 ml-Dreihalskolben gegeben, mit 800 ml konzentrierter Salpetersäure (65 %ig) und dann mit 25 ml einer 30 %igen Wasserstoffperoxid-Lösung versetzt. Das Reaktionsgemisch wird 12 Stunden lang auf etwa 80 °C erhitzt, wobei sich der schwarze Filterkuchen löst. Die erhaltene, auf Raumtemperatur abgekühlte schwarze Lösung wird über ein Glasfaserfilter filtriert, dann auf 60 °C erhitzt und mit 45 %iger KOH-Lösung versetzt, bis der pH-Wert etwa 8 beträgt. Es wird wiederum eine schwarze Suspension erhalten, die nach Abkühlen auf 40 °C über ein Blaubandfilter filtriert wird. Der Filterkuchen wird mit 11 I Wasser gewaschen; danach ist das Filtrat chlorid- und nitratfrei. Der schwarze Filterkuchen wird trockengesaugt und dann unter Zusatz von 200 ml Wasser in einen 2 I-Dreihalskolben überführt. Es werden 1000 ml konzentrierte Salpetersäure und dann 25 ml einer 30 %igen Wasserstoffperoxid-Lösung zugesetzt. Das Reaktionsgemisch wird wieder 12 Stunden lang auf etwa 80 °C erhitzt, wobei der schwarze Filterkuchen in Lösung geht. Die erhaltene, auf Raumtemperatur abgekühlte schwarze Lösung wird über ein Glasfaserfilter filtriert, dann auf 60 °C erwärmt und mit 45 %iger KOH-Lösung versetzt, bis der pH-Wert etwa 8 beträgt. Es wird wiederum eine schwarze Suspension erhalten, die nach Abkühlen auf 40 °C über ein Blaubandfilter filtriert wird. Der Filterkuchen wird mit 18 I Wasser gewaschen; danach ist das Filtrat chlorid-, nitrat- und kaliumfrei. Der Filterkuchen wird trockengesaugt. Der so gewonnene schwarze Feststoff wird mit 750 ml Eisessig in einen 2 I-Dreihalskolben überführt. Dann werden 850 ml Essigsäureanhydrid zugegeben, und die erhaltene Suspension wird 15 Stunden lang auf etwa 110 °C erwärmt. Die gebildete Lösung ist dunkelgrün und leicht getrübt; sie wird über ein Glasfaserfilter filtriert. Die filtrierte Lösung wird auf etwa 80 °C erhitzt, innerhalb von 1 Stunde mit 250 ml Wasser versetzt und anschließend im Rotationsverdampfer bei etwa. 80 °C zur Trockne eingedampft. Nachdem im Vakuumtrockenschrank bei 60 °C bis zur Gewichtskonstanz getrocknet wurde, liegt Iridiumacetat in Form dunkelgrüner, glänzender Kristalle vor; die Ausbeute beträgt 54 %.

## Patentansprüche

1. Verfahren zur Herstellung von Iridiumacetat, **dadurch gekennzeichnet, dass** aus einer wässerigen Lösung einer Iridiumchloroverbindung mit einer wässerigen Lösung eines Alkalimetallhydroxids, -carbonats oder -hydrogencarbonats Iridiumhydroxid gefällt, das gefällte Iridiumhydroxid abgetrennt und mit Essigsäure oder einem Essigsäure/Essigsäureanhydrid-Gemisch zu einer Iridiumacetat enthaltenden Lösung umgesetzt und das Iridiumacetat als Feststoff aus der Lösung isoliert wird.

2. Verfahren nach Anspruch 1, **dadurch gekennzeichnet, dass** das Iridiumhydroxid vor der Umsetzung mit Essigsäure oder dem Essigsäure/Essigsäureanhydrid-Gemisch umgefällt wird.

3. Verfahren nach Anspruch 2, **dadurch gekennzeichnet, dass** zur Umfällung das Iridiumhydroxid mit Salpetersäure oder mit Salpetersäure und Wasserstoffperoxid gelöst und aus der erzeugten Lösung durch Zugabe einer wässerigen Lösung eines Alkalimetallhydroxids, -carbonats oder -hydrogencarbonats erneut Iridiumhydroxid gefällt wird.

4. Verfahren nach Anspruch 3, **dadurch gekennzeichnet, dass** das Iridiumhydroxid unter Erwärmen auf bis zu 80 °C gelöst wird.

5. Verfahren nach einem der Ansprüche 1 bis 4, **dadurch gekennzeichnet, dass** als wässerige Lösung eines Alkalimetallhydroxids eine Kaliumhydroxid-Lösung eingesetzt wird.

6. Verfahren nach einem der Ansprüche 1 bis 5, **dadurch gekennzeichnet, dass** die Fällung von Iridiumhydroxid bei einem pH-Wert zwischen 6 und 9 und einer Temperatur von 50 - 90 °C erfolgt.

7. Verfahren nach einem der Ansprûche 1 bis 6, **dadurch gekennzeichnet, dass** als Iridiumchloroverbindung Iridium(III)-chlorid, Iridium(IV)-chlorid, Hexachloroiridium(III)-säure, Hexachloroiridium(IV)-säure oder ein Gemisch aus mindestens zwei dieser Verbindungen eingesetzt wird.

8. Verfahren nach einem der Ansprüche 1 bis 7, **dadurch gekennzeichnet, dass** eine wässerige Lösung der Iridiumchloroverbindung mit einem Iridium-Gehalt von 2 - 10 Gewichts-% eingesetzt wird.

9. Verfahren nach einem der Ansprüche 1 bis 8, **dadurch gekennzeichnet, dass** bei der Umsetzung des Iridiumhydroxids mit Essigsäure oder dem Essigsäure/Essigsäureanhydrid-Gemisch ein deutlich überstöchiometrisches Molverhältnis von Essigsäure zu Iridium eingestellt wird.

## Claims

1. Process for the preparation of iridium acetate, **characterized in that** iridium hydroxide is precipitated from an aqueous solution of a chloroiridium compound with an aqueous solution of an alkali metal hydroxide, alkali metal carbonate or alkali metal bicarbonate, the precipitated iridium hydroxide is separated off and is reacted with acetic acid or an acetic acid/acetic anhydride mixture to give a solution containing iridium acetate, and the iridium acetate is isolated as a solid from the solution.

2. Process according to Claim 1, **characterized in that** the iridium hydroxide is reprecipitated before the reaction with acetic acid or the acetic acid/acetic anhydride mixture.

3. Process according to Claim 2, **characterized in that**, for the reprecipitation, the iridium hydroxide is dissolved with nitric acid or with nitric acid and hydrogen peroxide and iridium hydroxide is precipitated again from the resulting solution by adding an aqueous solution of an alkali metal hydroxide, alkali metal carbonate or alkali metal bicarbonate.

4. Process according to Claim 3, **characterized in that** the iridium hydroxide is dissolved with heating to up to 80°C.

5. Process according to any of Claims 1 to 4, **characterized in that** a potassium hydroxide solution is used as the aqueous solution of an alkali metal hydroxide.

6. Process according to any of Claims 1 to 5, **characterized in that** the precipitation of iridium hydroxide is effected at a pH of from 6 to 9 and at a temperature of 50-90°C.

7. Process according to any of Claims 1 to 6, **characterized in that** the chloroiridium compound used is iridium(III) chloride, iridium(IV) chloride, hexachloroiridic(III) acid, hexachloroiridic(IV) acid or a mixture of at least two of these compounds.

8. Process according to any of Claims 1 to 7, **characterized in that** an aqueous solution of the chloroiridium compound having an iridium content of 2-10% by weight is used.

9. Process according to any of Claims 1 to 8, **characterized in that** a substantially superstoichiometric molar ratio of acetic acid to iridium is used in the reaction of the iridium hydroxide with acetic acid or with the acetic acid/acetic anhydride mixture.

## Revendications

1. Procédé de préparation d'acétate d'iridium, **caractérisé en ce qu'**on précipite de l'hydroxyde d'iridium à partir d'une solution aqueuse d'un composé chloré d'iridium avec une solution aqueuse d'un hydroxyde, d'un carbonate ou d'un hydrogénocarbonate de métal alcalin, qu'on sépare l'hydroxyde d'iridium précipité et qu'on le transforme avec de l'acide acétique ou un mélange d'acide acétique/anhydride d'acide acétique en une solution contenant de l'acétate d'iridium et qu'on isole l'acétate d'iridium de la solution sous forme de solide.

2. Procédé selon la revendication 1, **caractérisé en ce que** l'hydroxyde d'iridium est reprécipité avant la transformation avec l'acide acétique ou le mélange acide acétique/anhydride d'acide acétique.

3. Procédé selon la revendication 2, **caractérisé en ce qu'**en vue de la reprécipitation, on dissout l'hydroxyde d'iridium avec de l'acide nitrique ou avec de l'acide nitrique et du peroxyde d'hydrogène et qu'on précipite de nouveau l'hydroxyde d'iridium à partir de la solution produite par addition d'une solution aqueuse d'un hydroxyde, d'un carbonate ou d'un hydrogénocarbonate de métal alcalin.

4. Procédé selon la revendication 3, **caractérisé en ce que** l'hydroxyde d'iridium est dissous sous chauffage jusqu'à un maximum de 80°C.

5. Procédé selon l'une des revendications 1 à 4, **caractérisé en ce qu'**on utilise comme solution aqueuse d'un hydroxyde de métal alcalin une solution d'hydroxyde de potassium.

6. Procédé selon l'une des revendications 1 à 5, **caractérisé en ce que** la précipitation de l'hydroxyde d'iridium s'effectue à un pH compris entre 6 et 9 et à une température comprise entre 50 et 90°C.

7. Procédé selon l'une des revendications 1 à 6, **caractérisé en ce qu'**on utilise comme composé chloré d'iridium du chlorure d'iridium(III), du chlorure d'iridium(IV), de l'acide d'hexachloroiridium(III), de l'acide d'hexachloroiridium(IV) ou un mélange d'au moins deux de ces composés.

8. Procédé selon l'une des revendications 1 à 7, **caractérisé en ce qu'**on utilise une solution aqueuse du composé chloré d'iridium ayant une teneur en iridium de 2 à 10 % en poids.

9. Procédé selon l'une des revendications 1 à 8, **caractérisé en ce que** lors de la transformation de l'hydroxyde d'iridium avec de l'acide acétique ou le mélange acide acétique/anhydride d'acide acétique, on ajuste un rapport molaire nettement surstoechiométrique de l'acide acétique à l'iridium.
